# EUROPÄISCHE PATENTSCHRIFT

(11) **EP 2 510 953 B1**
(45) Veröffentlichungstag und Bekanntmachung des Hinweises auf die Patenterteilung: **18.06.2014**
(21) Anmeldenummer: 12176243.9
(22) Anmeldetag: 12.03.2010
(51) Int. Cl.: A61L 2/26, A61B 19/02

(54) **Sterilisierbehälter mit doppelter Abdichtung und Dichtelementträger**
Sterilisation container with double seals and seal support
Conteneur de stérilisation avec deux joint et support de joint

(30) Priorität: 18.03.2009 DE 102009014969
(43) Veröffentlichungstag der Anmeldung: 17.10.2012
(62) Teilanmeldung aus: 10156281.7
(73) Patentinhaber: AESCULAP AG, 78532 Tuttlingen (DE)
(72) Erfinder: OERTMANN, Friedrich, 78532 Tuttlingen (DE)
(74) Vertreter: Hoeger, Stellrecht & Partner Patentanwälte

(56) Entgegenhaltungen:
- DE-A1- 10 235 374
- DE-C1- 3 340 963
- US-A- 4 149 650
- US-A- 4 551 311

## Beschreibung

Die vorliegende Erfindung betrifft einen Sterilbehälter mit einem Unterteil und einem Oberteil zum Verschließen des Unterteils, welcher Sterilbehälter einen von dem Oberteil und dem Unterteil begrenzten Behälterinnenraum aufweist, wobei ein erstes, umlaufend am Oberteil und am Unterteil anliegendes Dichtelement vorgesehen ist zum Abdichten des Behälterinnenraums gegenüber einer Umgebung des Sterilbehälters.

Sterilbehälter der eingangs beschriebenen Art dienen in der Chirurgie insbesondere zum Sterilisieren und Lagern von chirurgischen Instrumenten oder Implantaten. Das Unterteil ist häufig wannenförmig ausgebildet und definiert so einen Aufnahmeraum für die Instrumente und/oder Implantate. Das erste Dichtungselement bildet einen Teil eines Dichtungssystems, um den Behälterinnerraum gegenüber einer Umgebung des Sterilbehälters abzudichten. Insbesondere verhindert das erste Dichtelement, dass auch bei längerer Lagerung Keime in den Behälterinnenraum gelangen können.

Ein Beispiel für einen Sterilbehälter der eingangs beschriebenen Art ist aus der DE 298 12 154 U1 bekannt. Das darin beschriebene Dichtelement weist zwei Dichtlippen auf.

Da Sterilbehälter der eingangs beschriebenen Art häufig über sehr viele Jahre hinweg im Einsatz sind, bleibt es nicht aus, dass die beispielsweise aus Aluminium gefertigten Ober- und Unterteile beschädigt oder mechanisch verformt werden. Auch das Dichtelement kann im Laufe der Jahre beschädigt werden. Immer dann, wenn auftretende Beschädigungen nicht rechtzeitig entdeckt werden, besteht potentiell die Gefahr, dass der Inhalt des Sterilbehälters bei der Lagerung durch Rekontamination unsteril wird.

Ein weiterer Sterilbehälter der eingangs beschriebenen Art ist aus der US 4,551,311 bekannt. Eine Vorrichtung, insbesondere zum Behandeln pharmazeutischer Behältnisse wie Vials oder Ampullen, ist in der DE 102 35 374 A1 beschrieben.

Es ist daher eine Aufgabe der vorliegenden Erfindung, einen Sterilbehälter der eingangs beschriebenen Art so zu verbessern, dass er auch bei einer sehr langen Nutzungszeit und gegebenenfalls auftretenden Beschädigungen den Behälterinnenraum dennoch optimal abdichtet.

Diese Aufgabe wird bei einem Sterilbehälter der eingangs beschriebenen Art erfindungsgemäß durch den kennzeichnenden Teil des Anspruchs 1 gelöst, wobei die Erfindung durch Anspruch 1 definiert ist.

Die zwei Dichtelemente des erfindungsgemäßen Sterilbehälters sind völlig unabhängig voneinander, und zwar räumlich und daher auch funktional, anders als dies bei einem Dichtelement mit zwei Dichtlippen der Fall ist. Zwei an einem Dichtelement ausgebildete Dichtlippen, wie sie zum Beispiel bei dem in der DE 298 12 154 U1 in Figur10 dargestellt sind, sind räumlich gerade nicht getrennt voneinander und wirken beide mit derselben Fläche des Sterilbehälters zusammen. Wird diese verformt, ist in der Regel eine Abdichtung durch die beiden Dichtlippen eines einzigen Dichtelements nicht mehr realisierbar. Dagegen wirken die beiden räumlich getrennten Dichtungen völlig unabhängig voneinander, so dass insgesamt die Gefahr einer Rekontamination des Behälterinnenraums und der darin gelagerten Teil ganz erheblich verringert wird, da

normalerweise auftretende Beschädigungen beziehungsweise Verformungen am Sterilbehälter und der Dichtung in der Regel lokal sehr begrenzt sind. Infolge einer solchen Beschädigung kann zwar möglicherweise das erste Dichtelement seine Funktion nicht mehr erfüllen, mit einer sehr großen Wahrscheinlichkeit ist jedoch die Funktion des zweiten Dichtelements, oder aber auch umgekehrt, hiervon nicht beeinträchtigt, so dass selbst bei Auftreten einer Beschädigung des Sterilbehälters, mindestens eines der beiden Dichtelemente seine Funktionsfähigkeit nicht einbüßt und den Behälterinnenraum in gewünschter Weise gegenüber der Umgebung des Sterilbehälters abdichtet, um das Eindringen von Keimen in den Behälterinnenraum zu vermeiden. Ein Abdichtungssystem des Sterilbehälters, welches die ersten und zweiten Dichtelemente umfasst, zeichnet sich somit durch eine redundante Konstruktion aus. Sowohl das erste als auch das zweite Dichtelement können beispielsweise in Form von Hohlkammerprofilen ausgebildet sein. Unter einem Hohlkammerprofil ist insbesondere ein Dichtelement zu verstehen, welches einen hohlen Dichtelementgrundkörper aufweist, welcher umlaufend, das heißt durchgängig, oder mindestens abschnittsweise hohl ausgebildet ist. Ein durchgängig hohler Dichtelementgrundkörper umfasst mindestens einen in sich ringförmig geschlossenen Hohlraum. Unter einer räumlichen Trennung der beiden Dichtelemente ist insbesondere zu verstehen, dass sie voneinander getrennt und nicht miteinander verbunden sind. Sie sind also separat ausgebildet. Günstigerweise wirken die beiden räumlich voneinander getrennten Dichtelementen mit unterschiedlichen Flächen des Sterilbehälters zusammen. Dies hat den Vorteil, dass dann, wenn eine der beiden Flächen beschädigt, insbesondere verformt wurde, der Behälterinnenraum immer noch ausreichend abgedichtet ist. Beispielsweise kann jeweils ein Dichtelement an einander gegenüberliegenden Flächen ausgebildet oder angeordnet sein, so dass es mit der jeweils anderen, gegenüberliegenden Fläche dichtend in Kontakt steht. Insbesondere kann es vorteilhaft sein, wenn die beiden räumlich voneinander getrennten Dichtelemente unterschiedlich ausgebildet sind. Denkbar sind hier zum Beispiel einerseits eine Flächendichtung und andererseits eine Dichtlippe, welche vorzugsweise an unterschiedlichen Flächen des Sterilbehälters eine Abdichtung bewirken. Gemäß der Erfindung ist vorgesehen, dass am Oberteil mindestens ein Dichtelementträger angeordnet oder ausgebildet ist, an welchem das zweite Dichtelement gehalten ist. Der Dichtelementträger kann insbesondere so individuell ausgebildet werden, dass er das Dichtelement optimal halten kann. Zum Beispiel kann er eine Querschnittsform oder überhaupt eine Form aufweisen, welche eine kraft- und/oder formschlüssige Verbindung mit dem zweiten Dichtelement ermöglicht. Vorteilhaft ist es, dass der mindestens eine Dichtelementträger entgegen der Wirkung einer Rückstelleinrichtung aus einer Grundstellung, in welcher er ohne äußere Krafteinwirkung am Oberteil gehalten ist, auslenkbar ist. Ein derart ausgebildeter Dichtelementträger kann beispielsweise auch eine Ventilfunktion übernehmen. Dies bedeutet, dass er bei einem beispielsweise wirkenden Über- oder Unterdruck in die eine oder in die andere Richtung ausgelenkt werden kann, um insbesondere einen Strömungspfad freizugeben, welcher eine sehr schnelle Be- und/oder Entlüftung des Behälterinnenraums ermöglicht.

Ein insbesondere zur Aufnahme von chirurgischen Instrumenten und/oder Implantaten hervorragend geeigneter Behälterinnenraum kann beispielsweise dadurch erhalten werden, dass das Unterteil eine Behälterwanne bildet.

Das Unterteil lässt sich auf einfache und sichere Weise verschließen, wenn das Oberteil einen Behälterdeckel bildet.

Vorzugsweise sind das erste und/oder das zweite Dichtelement am Oberteil angeordnet. Insbesondere können das erste und/oder das zweite Dichtelement am Oberteil gehalten sein. Insbesondere können sie beide am Oberteil befestigt sein. Wahlweise können aber auch nur das erste oder nur das zweite oder aber auch beide Dichtelemente am Oberteil in der beschriebenen Weise angeordnet sein.

Günstig kann es ferner sein, wenn das erste und/oder das zweite Dichtelement am Unterteil angeordnet sind. Dies bedeutet, dass das erste oder das zweite oder aber beide Dichtelemente am Unterteil angeordnet sein können. Insbesondere können sie am Unterteil gehalten oder dauerhaft an diesem festgelegt sein.

Eine besonders gute Abdichtung des Behälterinnenraums mit den ersten und zweiten Dichtelementen kann erreicht werden, wenn das erste und/oder das zweite Dichtelement mindestens eine Dichtkante und/oder mindestens eine abstehende Dichtlippe aufweisen. Das erste oder das zweite oder aber auch beide Dichtelemente können eine Dichtkante aufweisen, die an dem Behälterteil anliegt, an dem das jeweilige Dichtelement nicht gehalten beziehungsweise befestigt ist. Optional kann zusätzlich zur Dichtkante auch eine Dichtlippe vorgesehen sein. Alternativ wäre es auch denkbar, das erste oder das zweite oder aber auch beide Dichtelemente mit mindestens einer abstehenden Dichtlippe auszustatten.

Eine Abdichtung des Innenraums kann noch weiter verbessert werden, wenn das erste und/oder das zweite Dichtelement zwei oder mehr Dichtkanten und/oder zwei oder mehr abstehende Dichtlippen aufweisen. Zwei oder mehr Dichtkanten beziehungsweise zwei oder mehr Dichtlippen sind geeignet, um zwei oder mehr Barrieren auszubilden, welche beispielsweise von Keimen überwunden werden müssen, um von einer Umgebung des Sterilbehälters in den Behälterinnenraum zu gelangen. Allerdings haben zwei Dichtlippen oder Dichtkanten an einem einzigen Dichtelement im Vergleich zu zwei voneinander getrennten und unabhängig ausgebildeten und am Sterilbehälter angeordneten Dichtelementen den Nachteil, dass bereits eine lokale Beschädigung des Sterilbehälters zu einer Undichtigkeit führen kann, auch dann, wenn das Dichtelement zwei oder mehr Dichtlippen aufweist. Diese Gefahr wird durch die beiden voneinander räumlich getrennten Dichtelemente deutlich verringert.

Gemäß einer weiteren bevorzugten Ausführungsform der Erfindung kann vorgesehen sein, dass das erste und/oder das zweite Dichtelement am Oberteil oder am Unterteil gehalten ist und dass die mindestens eine Dichtkante oder Dichtlippe in Richtung auf den anderen Behälterteil hin weist und in einer Dichtstellung am anderen Behälterteil anliegt. Die Dichtstellung nimmt der Sterilbehälter insbesondere dann ein, wenn das Oberteil das Unterteil verschließt. Durch die Anordnung und Orientierung der ersten und/oder zweiten Dichtelemente an den den Sterilbehälter ausbildenden Teilen, kann auf einfache und sichere Weise eine optimale Abdichtung des Behälterinnenraums erreicht werden.

Günstig ist es, wenn das erste Dichtelement am Oberteil und wenn das zweite Dichtelement am Unterteil gehalten ist, so lässt sich auf besonders einfache Weise eine räumliche Trennung der Dichtelemente voneinander erreichen. Des Weiteren kann so auch eine Anordnung der Dichtelemente konstruktiv vereinfacht werden.

Um die Dichtigkeit des Sterilbehälters weiter zu verbessern, ist es günstig, wenn das erste und/oder das zweite Dichtelement aus einem elastisch verformbaren Material hergestellt sind. Auf diese Weise lassen sich Fertigungstoleranzen ausgleichen und auch etwaige leichte Beschädigungen und Verformungen am Ober- und/oder Unterteil ausgleichen.

Besonders einfach und kostengünstig herstellen lässt sich der Sterilbehälter, wenn das erste und/oder das zweite Dichtelement aus einem Kunststoff hergestellt sind. Insbesondere können die beiden Dichtelemente aus unterschiedlichen Materialien hergestellt sein, beispielsweise aus unterschiedlichen Kunststoffen .

Eine besonders hohe Elastizität der Dichtelemente kann sichergestellt werden, wenn das erste und/oder das zweite Dichtelement aus einem Gummimaterial hergestellt sind. Insbesondere aus elastischen Materialien hergestellte Dichtelemente eignen sich besonders gut für einen dauerhaften Einsatz, da sie in der Dichtstellung zwar verformt sein können, jedoch ihre ursprüngliche Form ganz oder nahezu ganz einnehmen, wenn das Oberteil und das Unterteil des Sterilbehälters wieder voneinander getrennt werden.

Besonders einfach und kostengünstig herstellen lässt sich der Sterilbehälter, wenn das erste und/oder das zweite Dichtelement aus einem Silikon oder einem Silikon enthaltenden Material hergestellt sind.

Vorteilhafterweise sind das erste und/oder das zweite Dichtelement einstückig ausgebildet. Eine einstückige Ausgestaltung mindestens eines der beiden Dichtelemente hat den Vorteil, dass es nahtfrei am Ober- beziehungsweise Unterteil angeordnet und an diesem gehalten werden kann. Zusätzliche Dichtstellen, die bei mehrteiligen Dichtelementen vorhanden sind, brauchen in diesem Fall weder berücksichtigt noch abgedichtet zu werden.

Der Aufbau des Sterilbehälters vereinfacht sich weiter, wenn das erste Dichtelement in Form eines Dichtringes ausgebildet ist. Selbstverständlich kann auch das zweite Dichtelement in Form eines Dichtringes ausgebildet sein.

Grundsätzlich sind viele Varianten denkbar, das erste und/oder das zweite Dichtelement am Sterilbehälter zu halten. Besonders einfach und sicher können die Dichtelemente am Sterilbehälter angeordnet werden, wenn das erste und/oder das zweite Dichtelement am Oberteil und/oder am Unterteil kraftund/oder formschlüssig gehalten sind. Beispielsweise kann mindestens eines der Dichtelemente in eine entsprechende Aussparung am Ober- oder Unterteil eingesetzt und darin im Klemmsitz gehalten werden. Umgekehrt kann auch ein Dichtelement so ausgebildet sein, dass ein Teil des Ober- beziehungsweise Unterteils in eine Aussparung oder Ausnehmung am Dichtelement eingreift.

Der Aufbau des Sterilbehälters kann weiter vereinfacht werden, wenn der mindestens eine Dichtelementträger in Form eines am Oberteil und/oder am Unterteil abstehenden Haltevorsprungs ausgebildet ist.

Vorteilhaft kann es ferner sein, wenn das erste und/oder das zweite Dichtelement am Oberteil und/oder am mindestens einen Dichtelementträger kraftund/oder formschlüssig gehalten sind. Insbesondere kann der mindestens eine Dichtelementträger hierfür eine Querschnittsform aufweisen, welche ein formschlüssiges in Eingriff Bringen mit einem oder zwei Dichtelementen ermöglicht.

Die Rückstelleinrichtung lässt sich besonders einfach ausbilden, wenn sie mindestens ein elastisches Rückstellglied umfasst.

Vorzugsweise ist das mindestens eine Rückstellglied in Form eines Federelements ausgebildet. Es kann in Form einer Schraubenfeder oder Blattfeder ausgebildet sein. Denkbar wären auch Tellerfedern. Selbstverständlich kann das mindestens eine Rückstellglied auch aus einem Kunststoffelement ausgebildet sein, das aus einem elastischen Kunststoff hergestellt ist. Beispielsweise kann es sich bei dem Kunststoff um Silikon handeln.

Vorzugsweise sind das erste und das zweite Dichtelement voneinander beabstandet am Ober- und/oder am Unterteil angeordnet. Dies bedeutet, dass sie vorzugsweise nicht aneinander anliegen oder einander berühren, wenn das Oberteil das Unterteil in einer Schließstellung des Sterilbehälters verschließt beziehungsweise in einer Dichtstellung abdichtet.

Besonders einfach anbringen lässt sich mindestens eines der beiden Dichtelemente am Sterilbehälter, wenn das erste und/oder das zweite Dichtelement an einer freien, umlaufenden Kante des Ober- und/oder des Unterteils gehalten sind. Beispielsweise kann dann eines der Dichtelemente mit einer entsprechenden Nut versehen sein, in welche die freie, umlaufende Kante in einer Verbindungsstellung, in welcher das Dichtelement am Ober- oder Unterteil gehalten ist, eingreifen kann.

Günstig ist es, wenn das erste und/oder das zweite Dichtelement in einer Dichtstellung an unterschiedlichen Flächen und/oder Teilen des Sterilbehälters dichtend anliegen. Dies hat insbesondere den Vorteil, dass dann, wenn eine der beiden Flächen oder eines der beiden Teile beschädigt, insbesondere verformt, wird, der Behälterinnenraum immer noch ausreichend abgedichtet ist. Beispielsweise kann jeweils ein Dichtelement an einander gegenüberliegenden Flächen ausgebildet oder angeordnet sein, so dass es mit der jeweils anderen, gegenüberliegenden Fläche dichtend in Kontakt steht.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht eines Sterilbehälters;
- Figur2:: eine Schnittansicht längs Linie 2-2 des Sterilbehälters in Figur 1;
- Figur 3:: eine Schnittansicht analog Figur 2 eines Sterilbehälters nicht gemäß der Erfindung;
- Figur 4:: eine Schnittansicht analog Figur 2 eines Ausführungsbeispiels eines Sterilbehälters gemäß der Erfindung.

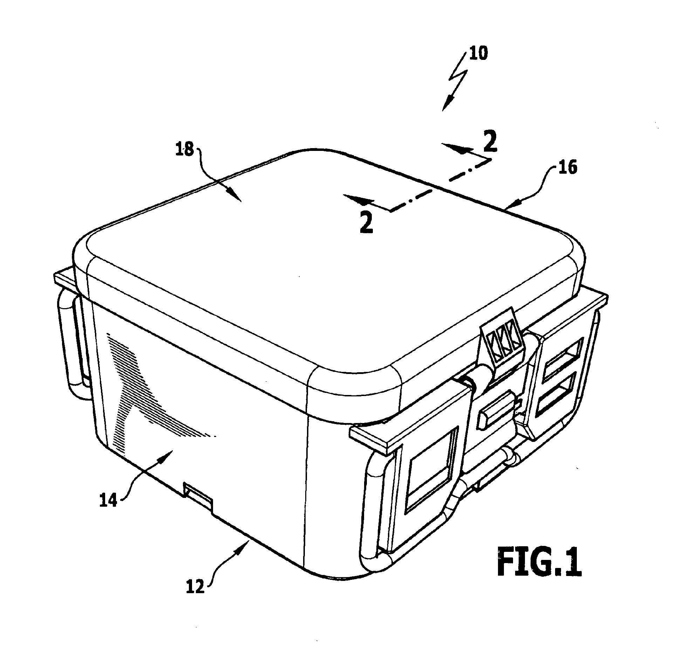

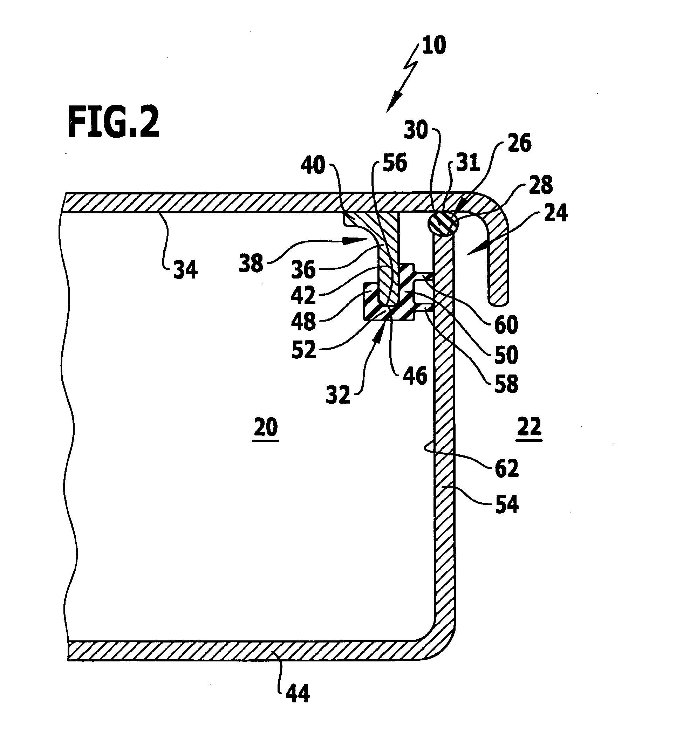

In Figur 1 ist ein insgesamt mit dem Bezugszeichen 10 versehener Sterilbehälter dargestellt, welcher ein insgesamt mit dem Bezugszeichen 12 versehenes Unterteil in Form einer Behälterwanne 14 und ein insgesamt mit dem Bezugszeichen 16 versehenes Oberteil in Form eines Deckels 18 umfasst. Die Behälterwanne 14 und der Deckel 18 sind so aufeinander abgestimmt, dass sie einen Behälterinnenraum 20, wie in Figur 2 schematisch dargestellt, begrenzen. Im Behälterinnenraum 20 können insbesondere chirurgische Instrumente und/oder Implantate steril, auch über einen längeren Zeitraum, gelagert werden.

Der Behälterinnenraum 20 ist gegenüber einer Umgebung 22 des Sterilbehälters 10 abgedichtet und zwar mit einem Dichtungssystem, welches insgesamt mit dem Bezugszeichen 24 bezeichnet ist. Das Dichtungssystem 24 umfasst ein erstes Dichtelement 26, welches am Deckel 18 derart befestigt ist, dass es dann, wenn der Deckel 18 die Behälterwanne 14 verschließt, an einer oberen Kante 28 der Behälterwanne 14 anliegt und so den Behälterinnenraum 20 gegenüber der Umgebung 22 abdichtet.Das erste Dichtelement 26 ist in Form eines einstückigen, in sich geschlossenen Dichtringes 30 ausgebildet, welcher in einem unbelasteten Zustand einen kreisförmigen oder im Wesentlichen kreisförmigen Querschnitt aufweist und somit eine Dichtkante 31 definiert. Das erste Dichtelement 26 ist am Oberteil 16 mit der Dichtkante 31 und am Unterteil 12 anliegend, so dass der Behälterinnenraum 20 in einer in den Figuren 1 und 2 dargestellten Dichtstellung gegenüber der Umgebung 22 abgedichtet ist.

Das Dichtungssystem 24 des Sterilbehälters 10 umfasst ein zweites, umlaufend am Oberteil 16 und am Unterteil 12 anliegendes Dichtelement 32 zum Abdichten des Behälterinnenraums 20 gegenüber der Umgebung 22 des Sterilbehälters 10. Das zweite Dichtelement 32 ist an einem von einer Unterseite 34 des Deckels 18 abstehenden, jedoch umlaufend ausgebildeten und im Querschnitt L-förmigen Haltevorsprungs 36 angeordnet, welches einen Dichtelementträger 38 bildet. Wie in Figur 2 dargestellt, umfasst der Haltevorsprung 36 einen kurzen Schenkel 40, welcher flächig an der Unterseite 34 befestigt ist, sowie einen senkrecht hierzu und damit auch senkrecht zur Unterseite 34 abstehenden längeren Schenkel 42, welcher eine in Richtung eines Bodens 44 der Behälterwanne 14 weisende freie, umlaufende Kante 46 definiert.

Das zweite Dichtelement 32 ist formschlüssig am Schenkel 42 gehalten. Zu diesem Zweck ist es im Querschnitt im Wesentlichen U-förmig mit zwei parallel zueinander verlaufenden Schenkeln 48 und 50, welche über einen Quersteg 52 miteinander verbunden sind. Der Quersteg 52 verläuft parallel zum Boden 44, die Schenkel 48 und 50 parallel zum Schenkel 42, wobei der kürzere Schenkel 48 auf einer von einer Seitenwand 54 der Behälterwanne 14 weg weisenden Seitenfläche des Schenkels 42 anliegt. Das zweite Dichtelement 32 definiert so eine umlaufende, nutförmige Ausnehmung 56, in welche der Schenkel 42 mit der Kante 46 einführbar ist, bis die Kante 46 am Quersteg 52 anschlägt. Vom längeren Schenkel 50 stehen senkrecht zwei umlaufende Dichtlippen 58 und 60 ab, welche an einer Innenfläche 62 der Seitenwand 54 anliegen.

Die Dichtelemente 26 und 32 bilden voneinander völlig unabhängige Abdichtungen des Behälterinnenraums 20 relativ zur Umgebung 22. Insbesondere dann, wenn beispielsweise der Deckel 18 beschädigt wird und das erste Dichtelement 26 keine umlaufende Abdichtung des Behälterinnenraums 20 mehr gewährleisten kann, kann die Abdichtung des Behälterinnenraums 20 unabhängig vom ersten Dichtelement 26 mittels des zweiten Dichtelements 32 sichergestellt werden, welches durch Anliegen der beiden Dichtlippen 58 und 60 an der Innenfläche 62 eine unabhängige Abdichtung mit zwei durch die Dichtlippen 58 und 60 definierte Sterilbarrieren gebildet wird.

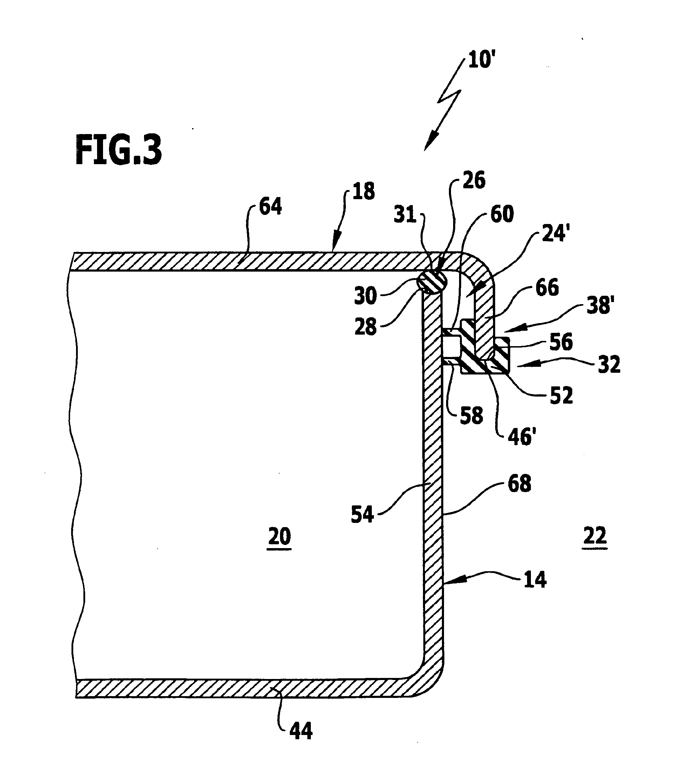

In Figur 3 ist ein Vergleichsbeispiel eines Sterilbehälters dargestellt und insgesamt mit dem Bezugszeichen 10' versehen. Es umfasst ein Dichtungssystem 24' mit zwei Dichtelementen 26 und 32, die identisch mit den Dichtelementen 26 und 32 des Sterilbehälters 10 ausgebildet sind. Das erste Dichtelement 26 ist wie oben im Zusammenhang mit Figur 2 des Sterilbehälters 10 dargestellt und beschrieben an der Kante 28 der Behälterwanne 14 festgelegt.

Das zweite Dichtelement 32 ist an einem umlaufenden Randabschnitt 66, welcher im Wesentlichen senkrecht von einer parallel zum Boden 44 in der Schließstellung verlaufenden Deckelwand 64 des Deckels 18 absteht, in analoger Weise gehalten wie am Schenkel 42 beim Sterilbehälter 10. Eine freie Kante 46' des Randabschnitts 66 ist in die umlaufende, nutförmige Ausnehmung des zweiten Dichtelements 32 eingeführt bis sie an dem Quersteg 52 anliegt. Die Dichtlippen 58 und 60 liegen, anders als beim Sterilbehälter 10, beim Sterilbehälter 10' an einer Außenfläche 68 der Seitenwand 54 an.

Beim Sterilbehälter 10' sind die beiden Dichtelemente 26 und 32 ebenfalls räumlich voneinander getrennt und bilden unabhängige Sterilbarrieren zur Abdichtung des Behälterinnenraums 20 von der Umgebung 22. Selbst bei der Beschädigung eines der beiden Dichtelemente 26 oder 32 bleibt der Behälterinnenraum 20 perfekt abgedichtet gegenüber der Umgebung 22.

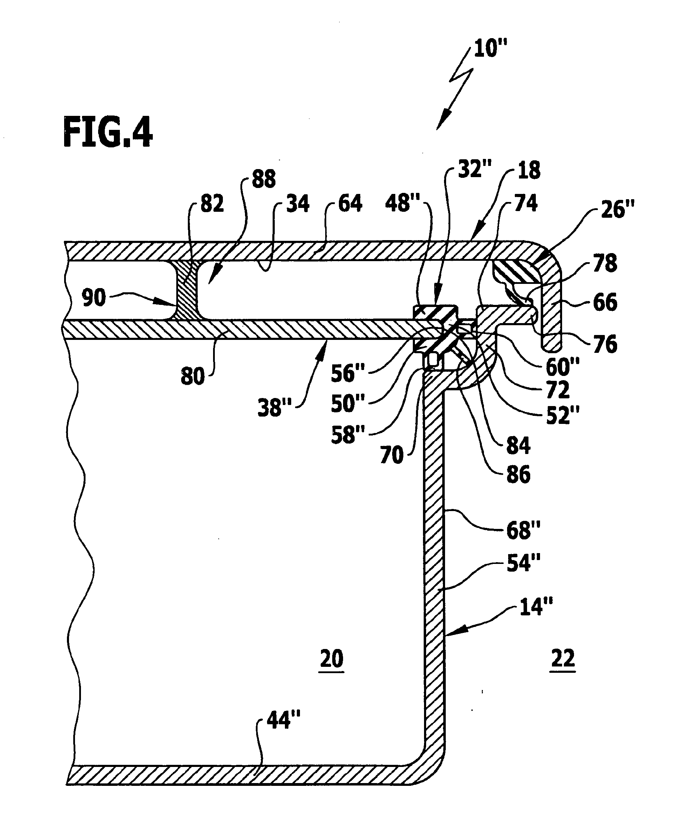

Ein Ausführungsbeispiel eines Sterilbehälters ist in Figur 4 teilweise im Schnitt dargestellt und insgesamt mit dem Bezugszeichen 10" versehen. Es umfasst eine Behälterwanne 14" und einen Deckel 18, welcher identisch mit den Deckeln 18 der Sterilbehälter 10 und 10' ausgebildet ist. An einem freien Ende 70 der Seitenwand 54" ist ein umlaufender, jedoch um 90° umgebogener Flanschabschnitt 72 ausgebildet, an dessen freiem Ende 74 ein quer zur Seitenwand 54" abstehender weiterer Flanschabschnitt 76 ausgebildet ist. Ein erstes umlaufendes Dichtelement 26" weist einen im Wesentlichen quaderförmigen Querschnitt auf und ist in einem Übergangsbereich zwischen der Deckelwand 64 und dem Randabschnitt 66 an der Unterseite 34 befestigt. Es weist eine in einer Grundstellung senkrecht abstehende und in einer in Figur 4 dargestellten Dichtstellung am Flanschabschnitt 76 anliegende Dichtlippe 78 auf, so dass der Behälterinnenraum 20 gegenüber der Umgebung 22 abgedichtet ist.

Eine Halteplatte 80 ist parallel zur Deckelwand 64 über elastische Halteelemente 82 mit der Unterseite 34 verbunden. Die Halteplatte 80 ist so bemessen, dass eine umlaufende Kante 84 nicht über die Außenfläche 68" der Seitenwand 54" vorsteht, wenn der Deckel 18 die Behälterwanne 14" verschließt.

Die Halteplatte 80 dient als Dichtelementträger 38" für ein zweites umlaufendes Dichtelement 32". Das Dichtelement 32" ist in Form eines im Querschnitt U-förmigen Dichtringes ausgebildet und mit einer umlaufenden, nutförmigen Ausnehmung 56" versehen, in welche die Kante 84 eingeführt ist. Die Schenkel 48" und 50" verlaufen parallel zur Halteplatte 80, der Quersteg 52" parallel zum Randabschnitt 66. Von dem Schenkel 50" und dem Quersteg 52" steht jeweils eine Dichtlippe 58" beziehungsweise 60" senkrecht und damit auch senkrecht relativ zueinander ab. Sie sind jeweils hohl und im Querschnitt im Wesentlichen quaderförmig und definieren so ein Hohlkammerprofil. Die Dichtlippe 48" liegt an einer in Richtung auf den Deckel 18 hin weisenden Kante des Endes 70 an, die Dichtlippe 60" an einer Innenfläche des Flanschabschnitts 72 im Bereich des Endes 74.

Im Übergangsbereich zwischen dem Schenkel 50" und dem Quersteg 52" steht eine weitere, jedoch einfache schmale Dichtlippe 86, die entsprechend der Dichtlippe 78 ausgebildet ist, in etwa symmetrisch zu beiden Dichtlippen 58" und 60" und somit etwa unter einem Winkel von 45° bezogen auf die Halteplatte 80 ab und liegt am Flanschabschnitt 52 im Bereich einer inneren Krümmung desselben an.

Das zweite Dichtelement 32" bildet somit eine vom ersten Dichtelement 26" räumlich getrennte Abdichtung des Behälterinnenraums 20 von der Umgebung 22. Beim Sterilbehälter 10" sind beide Dichtelemente 26" und 32" am Deckel 18 gehalten. Optional wäre es denkbar, das erste Dichtelemente 26" auch am Flanschabschnitt 76 festzulegen, so dass die Dichtlippe 78 an der Unterseite 34 der Deckelwand 64 anliegt.

Das Halteelement 82 kann insbesondere einen Teil einer Rückstelleinrichtung 88 bilden und in Form eines Rückstellglieds 90 ausgebildet sein. Eine solche Rückstelleinrichtung 88 ermöglicht es, den Dichtelementträger 38" entgegen der Wirkung die Rückstelleinrichtung 88 aus einer Grundstellung, in welcher er ohne äußere Krafteinwirkung am Deckel 18 gehalten ist, auszulenken. Das Rückstellglied 90 kann zu diesem Zweck insbesondere elastisch ausgebildet sein, und zwar in Form eines Federelements, zum Beispiel in Form einer Schraubenfeder. Das Rückstellglied 90 kann jedoch auch in Form eines elastischen Kunststoffteils, beispielsweise aus Silikon hergestellt, ausgebildet sein. Selbstverständlich können auch weitere Halteelemente 82 beziehungsweise Rückstellglieder 90 vorgesehen sein, um die Halteplatte 80 mit der Deckelwand 64 zu verbinden.

Die besondere Ausgestaltung des Sterilbehälters 10" gestattet es, den Deckel 18, insbesondere dessen Deckelwand 64, relativ zur Behälterwanne 14" zu bewegen, ohne dass eine Funktion des zweiten umlaufenden Dichtelements 32" beeinflusst wird. Mit anderen Worten: selbst dann, der Deckel 18 derart bewegt wird, dass keine Abdichtung mehr durch das erste umlaufende Dichtelement 26" gewährleistet ist, dichtet das zweite Dichtelement 32" den Behälterinnenraum 20 gegenüber der Umgebung 22 ab. Umgekehrt gilt dies entsprechend. So kann das erste Dichtelement 26" den Behälterinnenraum 20 trotzdem noch gegenüber der Umgebung 22 abdichten, selbst wenn eine Abdichtung durch das zweite Dichtelement 32" nicht mehr gewährleistet sein sollte.

Die Behälterwannen 14 und 14" sowie die Deckel 18 der oben beschriebenen Sterilbehälter 10, 10' und 10" sind vorzugsweise aus einem Metall, insbesondere aus Aluminium, hergestellt. Die somit überwiegend aus Metall hergestellten Sterilbehälter 10, 10' und 10" können durch die beschriebenen Dichtungssystems 24, 24' und 24" im Vergleich zu herkömmlichen Sterilbehältern die Gefahr ganz erheblich verringern, dass infolge einer Beschädigung oder Verformung der Behälterwannen 14 und 14" beziehungsweise des Deckels 18 eine Undichtigkeit beim Vorsehen nur eines Dichtelements entstehen kann. Da derartige Beschädigungen oder Verformungen des Sterilbehälters beziehungsweise eines Dichtelements in der Regel lokal sehr begrenzt sind, kann das zusätzliche Dichtelement die für einen dauerhaften Einsatz des Sterilbehälters erforderliche Abdichtung des Behälterinnenraums 20 gegenüber der Umgebung 22 und somit auch eine Keimfreiheit der im Behälterinnenraum 20 gelagerten Teile langfristig sicherstellen.

Die Dichtelemente 26, 26", 32 und 32" sind vorzugsweise aus einem elastisch verformbaren Material hergestellt, beispielsweise einem Kunststoff oder einem Gummimaterial. Denkbar ist es insbesondere, sie aus einem Silikon oder einem Silikon enthaltenden Material herzustellen. Das zweite Dichtelement 32 beziehungsweise 32" ist vorzugsweise ebenfalls einstückig ausgebildet. Es ist, wie insbesondere in den Figuren 2 bis 4 gut zu erkennen, räumlich getrennt vom ersten Dichtelement 26 beziehungsweise 26" am Sterilbehälter 10 angeordnet. Bei der oben beschriebenen Ausführungsform des Sterilbehälters 10 ist das erste Dichtelement 26 am Unterteil 12 gehalten, das zweite Dichtelement 32 am Oberteil 16. Optional wäre es denkbar, das erste Dichtelement 26 ebenfalls am Oberteil 16 zu halten, so dass beim Entfernen des Deckels 18 beide Dichtelemente 26 und 32 am Deckel 18 verbleiben und das Unterteil 12 dichtelementlos wäre.

## Patentansprüche

1. Sterilbehälter (10; 10'; 10") mit einem Unterteil (12) und einem Oberteil (16) zum Verschließen des Unterteils (12), welcher Sterilbehälter (10; 10'; 10") einen von dem Oberteil (16) und dem Unterteil (12) begrenzten Behälterinnenraum (20) aufweist, wobei ein erstes, umlaufend am Oberteil (16) und am Unterteil (12) anliegendes Dichtelement (26; 26") vorgesehen ist zum Abdichten des Behälterinnenraums (20) gegenüber einer Umgebung (22) des Sterilbehälters (10; 10'; 10"), **dadurch gekennzeichnet, dass** der Sterilbehälter ein zweites, umlaufend am Oberteil (16) und am Unterteil (12) anliegendes Dichtelement (32; 32") zum Abdichten des Behälterinnenraums (20) gegenüber der Umgebung (22) des Sterilbehälters (10; 10'; 10") umfasst, wobei das erste und das zweite Dichtelement (26, 32; 26", 32") voneinander räumlich getrennt am Sterilbehälter (10; 10'; 10") angeordnet sind, dass am Oberteil (16) mindestens ein Dichtelementträger (38; 38") angeordnet ist, an welchem das zweite Dichtelement (32; 32") gehalten ist, und dass der mindestens eine Dichtelementträger (38") entgegen der Wirkung einer Rückstelleinrichtung (88) aus einer Grundstellung, in welcher er ohne äußere Krafteinwirkung am Oberteil (16) gehalten ist, auslenkbar ist.

2. Sterilbehälter nach Anspruch 1, **dadurch gekennzeichnet, dass** der mindestens eine Dichtelementträger (38; 38") in Form eines am Oberteil (16) abstehenden Haltevorsprungs (36; 66; 80) ausgebildet ist.

3. Sterilbehälter nach Anspruch 1 oder 2, **dadurch gekennzeichnet, dass** das zweite Dichtelement (32; 32") am mindestens einen Dichtelementträger (38; 38") kraft- oder formschlüssig gehalten sind.

4. Sterilbehälter nach einem der voranstehenden Ansprüche, **dadurch gekennzeichnet, dass** die Rückstelleinrichtung (88) mindestens ein elastisches Rückstellglied (90) umfasst.

5. Sterilbehälter nach Anspruch 4, **dadurch gekennzeichnet, dass** das mindestens eine Rückstellglied (90) in Form eines Federelements ausgebildet ist, insbesondere in Form einer Schraubenfeder oder Blattfeder.

6. Sterilbehälter nach Anspruch 4, **dadurch gekennzeichnet, dass** das mindestens eine Rückstellglied (90) aus einem Kunststoffelement ausgebildet ist, das aus einem elastischen Kunststoff hergestellt ist, insbesondere aus Silikon.

7. Sterilbehälter nach einem der voranstehenden Ansprüche, **dadurch gekennzeichnet, dass** der Dichtelementträger (38; 38") derart angeordnet und ausgebildet ist, dass er bei einem wirkenden Über- oder Unterdruck in die eine oder in die andere Richtung ausgelenkt werden kann, um einen Strömungspfad freizugeben, welcher eine sehr schnelle Beoder Entlüftung des Behälterinnenraums (20) ermöglicht.

8. Sterilbehälter nach einem der voranstehenden Ansprüche, **dadurch gekennzeichnet, dass** der Dichtelementträger (38") in Form einer Halteplatte (80) ausgebildet ist, welcher parallel zu einer Deckelwand (64) über elastische Halteelemente (82) mit einer Unterseite (34) des Oberteils (16) verbunden ist.

9. Sterilbehälter nach Anspruch 8, **dadurch gekennzeichnet, dass** die Halteplatte (80) ist so bemessen, dass eine umlaufende Kante (84) derselben nicht über eine Außenfläche (68") einer Seitenwand (54") des Unterteils (12) vorsteht, wenn das Oberteil (16) das Unterteil (12) verschließt.

10. Sterilbehälter nach Anspruch 9, **dadurch gekennzeichnet, dass** das zweite Dichtelement (32") in Form eines im Querschnitt U-förmigen Dichtringes ausgebildet und mit einer umlaufenden, nutförmigen Ausnehmung (56") versehen ist, in welche die Kante (84) eingeführt ist.

11. Sterilbehälter nach einem der voranstehenden Ansprüche, **dadurch gekennzeichnet, dass** das erste und/oder das zweite Dichtelement (26, 32; 26", 32") mindestens eine Dichtkante oder mindestens eine abstehende Dichtlippe (58, 60; 58", 60", 78, 86) aufweisen.

12. Sterilbehälter nach einem der voranstehenden Ansprüche, **dadurch gekennzeichnet, dass** das erste und/oder das zweite Dichtelement (26, 32; 26", 32") einstückig ausgebildet sind.

13. Sterilbehälter nach einem der voranstehenden Ansprüche, **dadurch gekennzeichnet, dass** das erste und das zweite Dichtelement (26, 32; 26", 32") voneinander beabstandet am Oberteil (16) angeordnet sind.

14. Sterilbehälter nach einem der voranstehenden Ansprüche, **dadurch gekennzeichnet, dass** das erste Dichtelement (26, 32; 26", 32") an einer freien, umlaufenden Kante (46; 46'; 84) des Ober-und/oder des Unterteils (16, 12) gehalten ist.

15. Sterilbehälter nach einem der voranstehenden Ansprüche, **dadurch gekennzeichnet, dass** das erste und das zweite Dichtelement (26, 32; 26", 32") in einer Dichtstellung an unterschiedlichen Flächen (34, 62; 34, 68; 72, 76) oder Teilen (14, 18) des Sterilbehälters (10; 10'; 10") dichtend anliegen.

## Claims

1. Sterile container (10; 10'; 10") comprising a bottom part (12) and a top part (16) for closing the bottom part (12), said sterile container (10; 10'; 10") having a container interior (20) which is delimited by the top part (16) and the bottom part (12), a first sealing element (26; 26") lying circumferentially against the top part (16) and the bottom part (12) being provided for sealing off the container interior (20) from an environment (22) of the sterile container (10; 10'; 10"), **characterized in that** the sterile container comprises a second sealing element (32; 32") lying circumferentially against the top part (16) and the bottom part (12) for sealing off the container interior (20) from the environment (22) of the sterile container (10; 10'; 10"), the first and second sealing elements (26, 32; 26", 32") being arranged on the sterile container (10; 10'; 10") in spatially separate relation to each other, **in that** arranged on the top part (16) is at least one sealing element carrier (38; 38") on which the second sealing element (32; 32") is held, and **in that** the at least one sealing element carrier (38") can be deflected against the action of a resetting device (88) from a basic position in which it is held without the application of external force on the top part (16).

2. Sterile container in accordance with claim 1, **characterized in that** the at least one sealing element carrier (38; 38") is in the form of a holding projection (36; 66; 80) protruding from the top part (16).

3. Sterile container in accordance with claim 1 or 2, **characterized in that** the second sealing element (32; 32") is held with force locking or positive locking on the at least one sealing element carrier (38; 38").

4. Sterile container in accordance with any one of the preceding claims, **characterized in that** the resetting device (88) comprises at least one elastic resetting member (90).

5. Sterile container in accordance with claim 4, **characterized in that** the at least one resetting member (90) is in the form of a spring element, in particular, in the form of a helical spring or leaf spring.

6. Sterile container in accordance with claim 4, **characterized in that** the at least one resetting member (90) is formed by a plastic element which is made of an elastic plastic material, in particular, of silicone.

7. Sterile container in accordance with any one of the preceding claims, **characterized in that** the sealing element carrier (38; 38") is arranged and formed such that in the case of an acting positive or negative pressure it can be deflected in one or the other direction in order to open up a flow path which enables very rapid aeration or ventilation of the container interior (20).

8. Sterile container in accordance with any one of the preceding claims, **characterized in that** the sealing element carrier (38") is in the form of a holding plate (80) which is connected by elastic holding elements (82) to an underside (34) of the top part (16) parallel to a lid wall (64).

9. Sterile container in accordance with claim 8, **characterized in that** the holding plate (80) is of such dimensions that a circumferential edge (84) thereof does not protrude over an outer surface (68") of a side wall (54") of the bottom part (12) when the top part (16) closes the bottom part (12).

10. Sterile container in accordance with claim 9, **characterized in that** the second sealing element (32") is in the form of a sealing ring of U-shaped cross section and is provided with a circumferential, groove-shaped recess (56") in which the edge (84) is inserted.

11. Sterile container in accordance with any one of the preceding claims, **characterized in that** the first and/or the second sealing element (26, 32; 26", 32") comprise/comprises at least one sealing edge or at least one protruding sealing lip (58, 60; 58", 60", 78, 86).

12. Sterile container in accordance with any one of the preceding claims, **characterized in that** the first and/or the second sealing element (26, 32; 26", 32") is/are of one-piece configuration.

13. Sterile container in accordance with any one of the preceding claims, **characterized in that** the first and the second sealing elements (26, 32; 26", 32") are arranged in spaced relation to each other on the top part (16).

14. Sterile container in accordance with any one of the preceding claims, **characterized in that** the first sealing element (26, 32; 26", 32") is held at a free, circumferential edge (46; 46'; 84) of the top and/or the bottom part (16, 12).

15. Sterile container in accordance with any one of the preceding claims, **characterized in that** in a sealing position, the first and the second sealing elements (26, 32; 26", 32") lie in a sealing manner against different surfaces (34, 62; 34, 68; 72, 76) or parts (14, 18) of the sterile container (10; 10'; 10").

## Revendications

1. Récipient stérile (10; 10'; 10"), comprenant une partie inférieure (12) et une partie supérieure (16) destinée à fermer la partie inférieure (12), le récipient stérile (10; 10'; 10"") présentant un espace intérieur de récipient (20) délimité par la partie supérieure (16) et la partie inférieure (12), récipient dans lequel il est prévu un premier élément d'étanchéité (26; 26""), qui s'appuie de manière périphérique contre la partie supérieure (16) et contre la partie inférieure (12), et qui est destiné à rendre étanche l'espace intérieur de récipient (20) à l'encontre d'un environnement (22) du récipient stérile (10; 10'; 10"), **caractérisé en ce que** le récipient stérile comprend un deuxième élément d'étanchéité (32; 32"), qui s'appuie de manière périphérique contre la partie supérieure (16) et la partie inférieure (12), et qui est destiné à rendre étanche l'espace intérieur de récipient (20) par rapport à l'environnement (22) du récipient stérile (10; 10'; 10"), le premier et le deuxième élément d'étanchéité (26, 32; 26", 32") étant agencés de manière spatialement séparée sur le récipient stérile (10; 10'; 10"), **en ce que** sur la partie supérieure (16) est agencé au moins un support d'élément d'étanchéité (38; 38") sur lequel est maintenu le deuxième élément d'étanchéité (32; 32"), et **en ce que** ledit au moins un support d'élément d'étanchéité (38") peut être dévié, à l'encontre de l'action d'un dispositif de rappel (88), d'une position de base dans laquelle il est maintenu sur la partie supérieure (16) sans action de force extérieure.

2. Récipient stérile selon la revendication 1, **caractérisé en ce que** ledit au moins un support d'élément d'étanchéité (38; 38") est réalisé sous la forme d'une protubérance de maintien (36; 66; 80) faisant saillie de la partie supérieure (16).

3. Récipient stérile selon la revendication 1 ou la revendication 2, **caractérisé en ce que** le deuxième élément d'étanchéité (32; 32") est maintenu sur ledit au moins un support d'élément d'étanchéité (38; 38"), par adhérence ou par complémentarité de formes.

4. Récipient stérile selon l'une des revendications précédentes, **caractérisé en ce que** le dispositif de rappel (88) comprend au moins un organe de rappel élastique (90).

5. Récipient stérile selon la revendication 4, **caractérisé en ce que** ledit au moins un organe de rappel élastique (90) est réalisé sous la forme d'un élément de ressort, notamment sous la forme d'un ressort hélicoïdal ou d'un ressort à lame.

6. Récipient stérile selon la revendication 4, **caractérisé en ce que** ledit au moins un organe de rappel élastique (90) est réalisé par un élément en matière plastique, qui est fabriqué en une matière plastique élastique, notamment en silicone.

7. Récipient stérile selon l'une des revendications précédentes, **caractérisé en ce que** le support d'élément d'étanchéité (38; 38") est agencé et conçu de manière à pouvoir être dévié dans l'une ou dans l'autre direction sous l'effet d'une pression ou d'une dépression, en vue de libérer un parcours d'écoulement, qui permet une ventilation ou une purge d'air très rapide de l'espace intérieur de récipient (20).

8. Récipient stérile selon l'une des revendications précédentes, **caractérisé en ce que** le support d'élément d'étanchéité (38") est réalisé sous la forme d'une plaque de maintien (80), qui, parallèlement à une paroi de couvercle (64), est reliée à un côté inférieur (34) de la partie supérieure (16), par l'intermédiaire d'éléments de maintien élastiques (82).

9. Récipient stérile selon la revendication 8, **caractérisé en ce que** la plaque de maintien (80) est dimensionnée de manière telle, qu'un bord périphérique (84) de celle-ci ne fasse pas saillie au-delà d'une surface extérieure (68") d'une paroi latérale (54") de la partie inférieure (12), lorsque la partie supérieure (16) ferme la partie inférieure (12).

10. Récipient stérile selon la revendication 9, **caractérisé en ce que** le deuxième élément d'étanchéité (32") est réalisé sous la forme d'un anneau d'étanchéité en forme de U en section transversale, et est pourvu d'un évidement périphérique (56") en forme de rainure, dans laquelle est inséré le bord (84).

11. Récipient stérile selon l'une des revendications précédentes, **caractérisé en ce que** le premier et/ou le deuxième élément d'étanchéité (26, 32; 26", 32") présente au moins une arête d'étanchéité ou au moins une lèvre d'étanchéité (58, 60; 58", 60", 78, 86) en saillie.

12. Récipient stérile selon l'une des revendications précédentes, **caractérisé en ce que** le premier et/ou le deuxième élément d'étanchéité (26, 32; 26" , 32") sont réalisés en une seule partie.

13. Récipient stérile selon l'une des revendications précédentes, **caractérisé en ce que** le premier et le deuxième élément d'étanchéité (26, 32; 26", 32") sont agencés de manière espacée l'un de l'autre sur la partie supérieure (16).

14. Récipient stérile selon l'une des revendications précédentes, **caractérisé en ce que** le premier élément d'étanchéité (26, 32; 26", 32") est maintenu sur un bord périphérique libre (46; 46'; 84) de la partie supérieure et/ou inférieure (16, 12).

15. Récipient stérile selon l'une des revendications précédentes, **caractérisé en ce que** dans une position d'étanchéité, le premier et le deuxième élément d'étanchéité (26, 32; 26", 32") s'appuient de manière étanche sur des surfaces différentes (34, 62; 34, 68; 72, 76) ou des parties différentes (14, 18) du récipient stérile (10; 10'; 10").
